# EUROPEAN PATENT SPECIFICATION

(11) **EP 3 463 550 B1**
(45) Date of publication and mention of the grant of the patent: **27.03.2024**
(21) Application number: 17805984.6
(22) Date of filing: 31.05.2017
(51) Int. Cl.: A61N 1/36, A61B 5/00

(54) **NERVE STIMULATION APPARATUS AND METHOD**
VORRICHTUNG UND VERFAHREN ZUR NERVENSTIMULATION
APPAREIL ET MÉTHODE DE STIMULATION DU NERF

(30) Priority: 31.05.2016 US 201662343405 P
(43) Date of publication of application: 10.04.2019
(73) Proprietor: Lab Schöpfergeist AG, 9496 Balzers (LI)
(72) Inventor: LABUSCHAGNE, Chene, 7600 Western Cape (ZA); DUFFY, Kevin, Lee On Solent Hampshire PO13 9NR (GB); HENRY, Terrence Howard, 7460 Cape Town (ZA)
(74) Representative: Engstle, Verena
(86) International application number: PCT/IB2017/053198
(87) International publication number: WO 2017/208167

(56) References cited:
- WO-A1-2013/074809
- WO-A1-2015/187712
- US-A1- 2007 179 534
- US-A1- 2009 281 594
- US-A1- 2015 273 215
- US-A1- 2015 321 000
- US-A1- 2015 321 000
- FIELDS, R.D.: 'Amping Up Brain Function: Transcranial Stimulation Shows Promise in Speeding Up Learning' SCIENTIFIC AMERICAN 25 November 2011, XP055444577

## Description

### FIELD OF THE INVENTION

THIS INVENTION relates to a system for and method of stimulating nerves in the arm, wrist or hand, and in particular comprising an apparatus for the neurostimulation of the peripheral nerves of a user, including, but not limited to, the ulnar and/or median nerves.

In an embodiment, the apparatus may take the form of a portable, non-invasive module arranged to stimulate the median and/or ulnar nerves using electricity, light, sound, magnetic field, vibration or pressure, proximate the arm, wrist or hand of a user, which may either take the form of a standalone band or clasp, or which may be integrated into a fitness tracker band, watch or smartwatch.

The potential users of the apparatus (and related methodologies) include individual consumers and/or patients, with a view to improving non-medical indications and medical indications.

The non-medical indications include, but are not limited to, sports performance and endurance, including addressing fatigue, alertness and motorskill development. The non-medical indications may further include, but again are not limited to, improving cognitive performance, such as learning, reading, attention or multitasking, weight loss and jet lag, as well as mindfulness.

The medical indications, the treatment of which is considered therapeutical and is thus not under the scope of the non-therapeutic method defined in claim 13, include, but are not limited to, improving ADHD, depression, epilepsy, insomnia, migraine, anxiety, acute and chronic pain, cardiovascular disorders, movement disorders and functional restoration.

### BACKGROUND OF THE INVENTION

The nervous system consists of the central nervous system and peripheral nervous system. The function of the peripheral nervous system (PNS) is to connect the central nervous system to the limbs and organs.

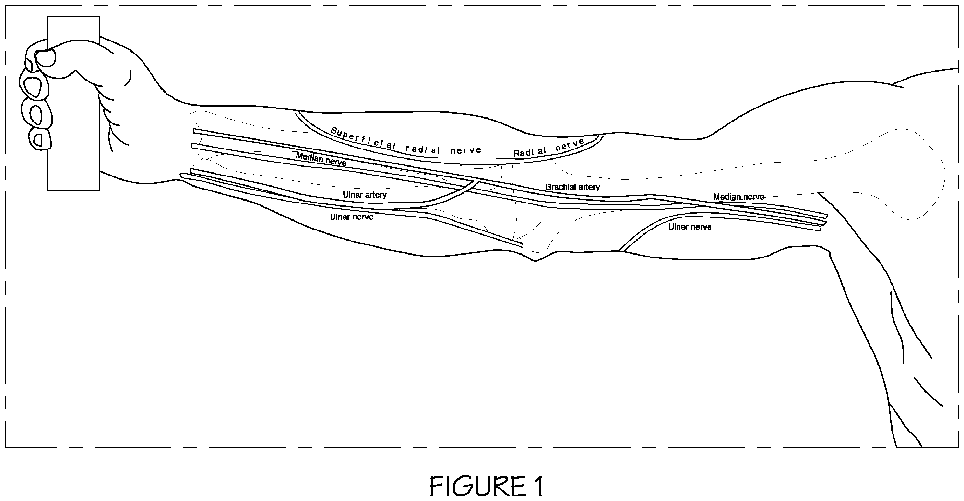

Various spinal nerves contribute to form nerve plexi-networks of interconnecting nerves. The brachial plexus is one of a few major plexi, and is fed into from five of the 31 spinal nerves. Extending from the brachial plexus into the human forearm are the median and ulnar nerves, as shown in Figure 1. The median and ulnar nerves form conduits to the brain and autonomic nervous system via the brachial plexus and spinal nerves. Stimulation of these nerves can influence motor/sensory brain functions, as well as the autonomic nervous system.

US2015/0321000 discloses a peripheral nerve stimulator to stimulate a peripheral nerve to treat essential tremor, Parkinson tremor, and other forms of tremor. The peripheral nerve stimulator can be either a non-invasive surface stimulator or an implanted stimulator. The stimulation can be electrical, mechanical, or chemical, and can be delivered using either an open loop system or a closed loop system with feedback.

WO2013/074809 relates to an apparatus for Transcutaneous Electrical Nerve Stimulation (TENS) delivering electrical currents across the intact skin of a patient via electrodes so as to provide symptomatic relief of chronic pain.

WO2015/187712 discloses a peripheral nerve stimulator to stimulate a peripheral nerve to treat essential tremor, Parkinsonian tremor, and other forms of tremor. The stimulator can have electrodes that are placed circumferentially around the patient's wrist or arm. Specific nerves in the wrist or arm can be targeted by appropriate spacing of the electrodes. Positioning the electrodes on generally opposing sides of the target nerve can result in improved stimulation of the nerve. The stimulation pattern may alternate between the nerves. Improved stimulation algorithms can incorporate tremor feedback, external data, predictive adaptation, and long-term monitoring data.

### SUMMARY OF THE INVENTION

The inventive system is defined in independent claim 1 and the inventive non-therapeutic method is defined in claim 13. Their dependent claims relate to further preferred embodiments of the invention.

### SUMMARY OF THE DISCLOSURE

In broad terms, described herein is a nerve stimulator, and in particular a non-invasive nerve stimulator for peripheral nerves, such as the ulnar and/or median nerves, which may be fitted proximate the left and/or right arm, wrist or hand of a user, to stimulate the median and/or ulnar nerves using electricity, light, sound, magnetic field, vibration or pressure, or any combination of these stimuli, the apparatus taking the form of a standalone band or clasp, or forming part of a fitness tracker band, watch or smartwatch. The nerve stimulator, or a plurality of stimulators, may be fitted so as to stimulate the user's left arm and/or wrist and/or hand, the user's right arm and/or wrist and/or hand, or the user's left and right arm and/or wrist and/or hand.

In slightly more detail, the nerve stimulator comprises a stimulus generator, to generate any or a combination of the stimuli mentioned above, an applicator to apply the generated stimulus to the arm, wrist or hand of the user, and a controller to control the operation of the stimulus generator and the applicator. In some cases, for stimuli such as light, sound, magnetic field, vibration or pressure, the generator and applicator may be combined into a single component.

In an embodiment, the applicator comprises at least one stimulus interface, which would take various forms, depending on the nature of the stimulus, to apply the stimulus to the user's skin, typically proximate to the anterior or ventral side of the user's arm, wrist or hand.

In an embodiment, the stimulus may be applied either as a continuous waveform, including square, rectangular, sinusoidal or triangular waveforms, or as a series of pulses.

In an embodiment, and depending on the application, the stimulator may include user biometric measuring sensors, which may be embodied in or proximate the applicator (but need not be necessarily), to monitor and determine physiological parameters associated with the user, including any one of, but not limited to, heart rate (HR), heart rate variability (HRV), temperature, SPO2, GSR (galvanic skin response) and inertial measurement.

In an embodiment, the controller may include a user biometric measuring module in communication with the user biometric measuring sensors.

In an embodiment, the controller may include a GSR (galvanic skin response) detection module in communication with a GSR sensor, to measure the electrical conductance of the user's skin and/or a user biometric measuring module.

In an embodiment, controller may include an inertial measurement (IMU) module in communication with a IMU sensor to determine whether the user is engaged in athletic activity.

In some embodiments, the user biometric measuring sensors, for monitoring and determining the physiological parameters associated with the user, and the stimulus interface are spaced apart or segmented by at least 30 degrees around the circumference of the user's arm, wrist or hand.

In an embodiment, the controller includes a stimulus controller module to control the applied stimulation in accordance with a stimulation profile, the stimulation profile defining the applied stimulus in terms of duration and/or frequency and/or intensity/amplitude and/or width.

In an embodiment, and again depending on the application, the stimulator may include a communication module to facilitate communication and data transfer between the nerve stimulator and an external/remote device, including the user's mobile device (i.e. smartphone, tablet or smartwatch) and/or a remote server on the cloud. It is envisaged that the data being transferred from the stimulator may include information regarding the stimulus being applied, including duration, frequency, strength etc. and/or the physiological parameters determined by the biometric measuring sensors. Conversely, it is envisaged that the data being transferred to the stimulator from the external/remote device may include information regarding a variation to the stimulus to be applied (i.e. to implement an amended or adjusted stimulation profile), which the controller may then implement, via the stimulus controller module, in conjunction with the generator and applicator.

In an embodiment, a battery and related battery charger may be connected to the applicator.

### Electrical Stimulus

In the case in which the stimulus is an electrical stimulus, the stimulus generator includes a high voltage generator and a current limiter to generate the electrical stimulus for application via a switching matrix.

In an embodiment, the electrical stimulus comprises a series of electrical pulses, with the stimulus controller module including a switch control module and a pulse control module to control switching of the electrical stimulus, via the switching matrix, in accordance with the stimulation profile.

In an embodiment, the stimulus interface includes at least one switching arrangement connected to the switching matrix to control the operation of the stimulus interface.

In an embodiment, the battery and related battery charger are connected to the applicator, and in particular to each switching arrangement associated with each stimulus interface.

In a first embodiment, the stimulus interface includes at least one electrode pair to send current through the user's tissue in order to stimulate the relevant nerve/s.

In an embodiment, at least one electrode pair has a dual function to enable the electrode pair to provide and receive electrical current.

In an embodiment, the electrode pairs may be arranged into a predetermined configuration to allow targeted stimulation of specific areas of the nervous system. More specifically, a configuration of electrode pairs running parallel may be applied to establish proximity for targeting the ulnar and/or the median nerves, which run alongside the length of the forearm. In one embodiment, two electrode pairs may be provided in a criss-cross pattern to target the ulnar and/or median nerves.

In the case of a series of electrical pulses, each stimulation pulse may comprise a pulse of a single frequency, or may comprise a pulse comprising a combination of two or more pulses of the same or different frequencies.

In an envisaged application, the pulse stimulation profile may have the following parameters:

| | |
|---|---|
| Frequency range: | 0.1 Hz to 1000 Hz |
| Intensity: | 100 uA to 10 mA |
| Pulsewidth: | 1 uS to 100 mS |

In a particular embodiment, the stimulation may comprise the sum of two or more frequencies, for example a slow oscillating waveform, having a frequency of around 1 Hz, a medium oscillating waveform superimposed on top of the slow oscillating waveform having a frequency of around 15 Hz, and a high oscillating waveform superimposed on top of the slow waveform pulse and the medium oscillating waveform, having a frequency of between 30 Hz and 50 Hz.

### Magnetic Stimulus

In the case in which the stimulus is a magnetic stimulus, the stimulus generator includes a time varying magnetic field generator and related stimulus interface to induce an electrical current in the user's tissue, in order to stimulate the nerve/s.

### Light Stimulus

In the case in which the stimulus is a light stimulus, the stimulus generator includes a light source, such as a light-emitting diode (LED), or a laser, in order to stimulate the nerve/s. Unlike electrical stimulation, which spreads through tissue and cannot readily be focused, light stimulation offers the advantage of being able to be pinpointed.

### Sound Stimulus

In the case in which the stimulus is sound, the stimulus generator includes a sound generator in order to stimulate the nerve/s.

### Vibration Stimulus

In the case in which the stimulus is a vibration stimulus, the stimulus generator includes a vibration generator, in order to stimulate the nerve/s.

### Pressure Stimulus

In the case in which the stimulus is a pressure stimulus, the stimulus generator includes a vibration generator, in order to stimulate the nerve/s.

In an embodiment, the nerve stimulator may take the form of a portable, wearable stimulator, which may be embodied and/or housed and/or accommodated and/or integrated within and/or secured to (with a clip, for example) a fitness tracker band, clasp, patch, strap, or sleeve or conventional watch. In one version, and with particular reference to watches (typically, upper end, luxury watches) that make use of so-called butterfly clasps comprising a central, curved clipping body, to which adjacent flexible strap portions are hingedly fitted, the nerve stimulator may be integrally fitted, or removably fittable, to the clipping body.

In a first application, the nerve stimulator may take the form of a portable, wearable stimulator, which may be coupled to a smartwatch to form an integral device.

In one particular embodiment, a smartwatch comprises a watch computing device fitted to a strap, typically positioned on the dorsal side of the user's wrist, with the ventral side of the strap comprising the nerve stimulator of the present invention. Although they are separate and distinct components, the communication module of the nerve stimulator enables communications and/or the transfer of data and information between the watch computing device and the nerve stimulator, typically wirelessly. In this application, the watch computing device and the nerve stimulator may be arranged diametrically opposite each other on the strap.

The communication module of the nerve stimulator may be any suitable communication component for both transmitting stimulation data to and receiving biometric data from the processor located on the dorsal side of the wrist. In addition, the pulse control module may also include controller logic to trigger electrical stimulation pulse profiles based on received biometric data from the smart watch.

In a second application, the stimulus controller module applies a stimulation profile based on the physiological parameters received by the user biometric measuring module from the user biometric measuring sensors and/or an indication from the GSR detection module and/or IMU module, so as to define a closed loop bio-feedback arrangement.

In this embodiment, the nerve stimulator may take the form of a wearable band, patch, or strap, of the type described above, which may be worn on the user's arm, wrist or hand.

In one version, the stimulus controller module may compare the received physiological parameters to a predetermined level or profile, and select an alternative stimulation profile (or adjust the existing stimulation profile being used).

The stimulus controller module continues to monitor the physiological parameters, and once the predetermined level or profile of the physiological parameters is achieved, yet another stimulation profile may be selected and applied to maintain the predetermined level or profile of the physiological parameters (or the stimulation may be stopped).

Thus, in a related method, the invention extends to a method of operating a nerve stimulator of the type defined above, the method comprising:
receiving physiological parameters associated with a user;
comparing the received physiological parameters to a predetermined level or profile; and
selecting an alternative stimulation profile, or adjust the existing stimulation
profile being used, accordingly.

The method includes continuing to monitor the physiological parameters, and once the predetermined level or profile of the physiological parameters is achieved, the method includes selecting another stimulation profile to maintain the predetermined level or profile of the physiological parameters (or stopping the stimulation).

According to the invention, in a third application, the nerve stimulator is used in conjunction with an interactive computer cognitive training application, such as a language or education software running on a training computer, with which the user interacts. In this inventive application, a training processing module is provided to co ordinate the application of the stimulus with particular activities or learning elements that the user must engage with, and/or at predetermined times during the training.

The training processing module monitors the user's performance during the training, and instructs the stimulus controller module to select a stimulation profile (or select another stimulation profile, if one is already being used.) The training processing module continues to monitor the user's performance, and once a satisfactory performance level has been achieved, yet another stimulation profile may be selected and applied to maintain the user's performance (or the stimulation may be stopped).

In this embodiment, the nerve stimulator may take the form of a wearable band, patch, or strap, of the type described above, which may be worn on the user's arm, wrist or hand, the nerve stimulator being connected to (or at least in communication with) the training processing module (via the communication module and related communication component), which in turn is connected to (or at least in communication with) the training computer.

In a fourth application, the nerve stimulator may be used in conjunction with a visualizing device running on a visualization computer, which the user may interact with to view corresponding anatomical movements during stimulation of the user's median and/or ulnar nerves.

In this application, a visualization module is provided to co ordinate, typically in tandem, the application of the stimulation to the median and/or ulnar nerves with the display on the visualizing device of the movement of a muscle or muscle group associated with the median and/ulnar nerve. The visualizing device may take the form of a virtual reality or ocular feedback device. In an embodiment, the extent and nature of the virtually depicted activity or movement will coincide with the amount of stimulation provided.

In this embodiment, the nerve stimulator may take the form of a wearable band, patch, or strap, of the type described above, which may be worn on the user's arm, wrist or hand, the nerve stimulator being connected to (or at least in communication with) the visualization module (via the communication module and related communication component), which in turn is connected to (or at least in communication with) the visualization computer.

### BRIEF DESCRIPTION OF THE DRAWINGS

- **Figure 1**: shows an anatomical illustration of the median and ulnar nerves;
- **Figure 2**: shows a schematic view of a nerve stimulator, according to an embodiment in which the stimulator takes the form of a wrist band;
- **Figure 3**: shows a schematic view of a nerve stimulator, according to an embodiment in which the stimulator is coupled to a smartwatch;
- **Figure 4**: shows a high level schematic block diagram of some of the possible components of the nerve stimulator;

- **Figure 5**: shows a schematic block diagram of one possible version of the nerve stimulator, in which the nerve stimulator makes use of electrical stimulation;
- **Figure 6**: shows an example pulse stimulation waveform of the type that may be generated by the nerve stimulator;

- **Figure 7**: shows an alternative pulse stimulation waveform of the type that may be generated by the nerve stimulator;

- **Figure 8**: shows a schematic diagram of the nerve stimulator used in conjunction with an interactive computer cognitive training application, according to the invention;
- **Figure 9**: shows a schematic diagram of the nerve stimulator used in conjunction with a visualization application; and
- **Figure 10**: shows a schematic flow chart representing a method of operating a non-invasive nerve stimulator for peripheral nerves.

### DETAILED DESCRIPTION OF THE INVENTION

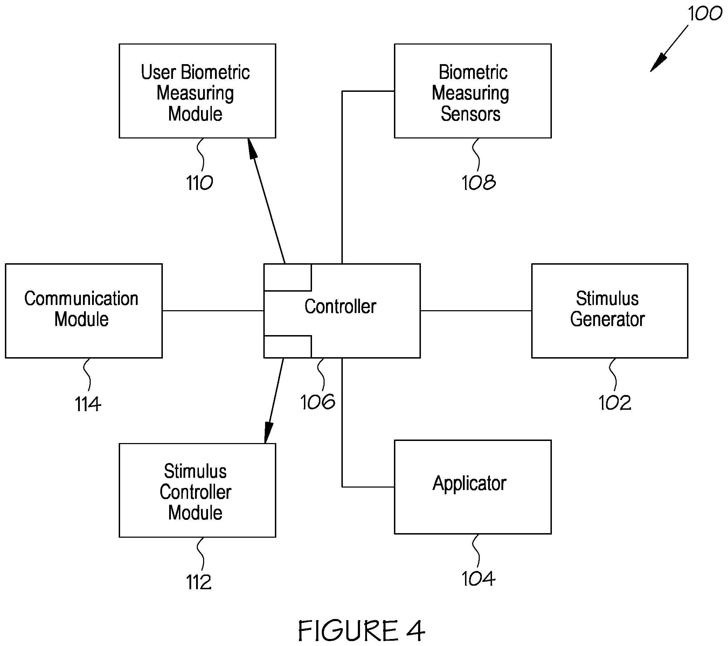

Referring first to Figure 4, in broad terms, a nerve stimulator 100 is provided, and in particular a non-invasive nerve stimulator for peripheral nerves, such as the ulnar and/or median nerves, which may be fitted proximate the arm, wrist or hand of a user, to stimulate the median and/or ulnar nerves using electricity, light, sound, magnetic field, vibration or pressure, or any combination of these stimuli. The stimulator 100 may take the form of a standalone band or clasp, or forming part of a fitness tracker band, watch or smartwatch.

The nerve stimulator 100 may comprise a stimulus generator 102, to generate any or a combination of the stimuli mentioned above, an applicator 104 to apply the generated stimulus to the arm, wrist or hand of the user, and a controller 106 to control the operation of the stimulus generator 102 and the applicator 104. In some cases, for stimuli such as light, sound, magnetic field, vibration or pressure, the generator 102 and applicator 104 may be combined into a single component.

In an embodiment, the applicator 104 comprises at least one stimulus interface, which could take various forms, depending on the nature of the stimulus, to apply the stimulus to the user's skin, typically proximate to the anterior or ventral side of the user's arm, wrist or hand.

The stimulus may be applied either as a continuous waveform, including square, rectangular, sinusoidal or triangular waveforms, or as a series of pulses.

In an embodiment, and depending on the application, the stimulator 100 may include user biometric measuring sensors 108, which may be embodied in or proximate the applicator 104 (but need not be necessarily), to monitor and determine physiological parameters associated with the user, including any one of, but not limited to, heart rate (HR), heart rate variability (HRV), temperature, SPO2, GSR (galvanic skin response) and inertial measurement.

In an embodiment, the controller 106 may include a user biometric measuring module 110 in communication with the user biometric measuring sensors 108. The controller 106 may include a GSR (galvanic skin response) detection module in communication with a GSR sensor, to measure the electrical conductance of the user's skin and/or a user biometric measuring module. In addition, the controller 106 may include an inertial measurement (IMU) module in communication with an IMU sensor to determine whether the user is engaged in athletic activity.

In some embodiments, the user biometric measuring sensors 108, for monitoring and determining the physiological parameters associated with the user, and the stimulus interface are spaced apart or segmented by at least 30 degrees around the circumference of the user's arm, wrist or hand.

In an embodiment, the controller 106 includes a stimulus controller module 112 to control the applied stimulation in accordance with a stimulation profile, the stimulation profile defining the applied stimulus in terms of duration and/or frequency and/or intensity/amplitude and/or width.

In an embodiment, and again depending on the application, the stimulator 100 may include a communication module 114 to facilitate communication and data transfer between the nerve stimulator 100 and an external/remote device, including the user's mobile device (i.e. smartphone, tablet or smartwatch) and/or a remote server on the cloud.

It is envisaged that the data being transferred from the stimulator 100 may include information regarding the stimulus being applied, including duration, frequency, strength etc. and/or the physiological parameters determined by the biometric measuring sensors 108.

Conversely, it is envisaged that the data being transferred to the stimulator 100 from the external/remote device may include information regarding a variation to the stimulus to be applied (i.e. to implement an amended or adjusted stimulation profile), which the controller 106 may then implement, via the stimulus controller module 112, in conjunction with the generator 102 and applicator 104.

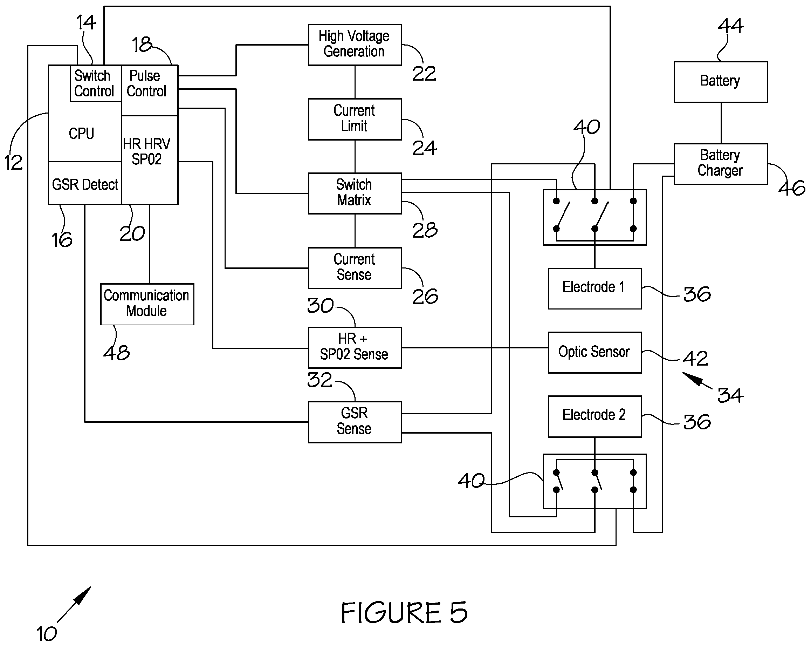

Turning now to Figure 5, a nerve stimulator 10, and in particular a non-invasive nerve stimulator 10 for electrically stimulating a user's ulnar and/or median nerves, is provided. This may, however, be applied to all peripheral nerves.

The stimulator 10 comprises a controller 12 comprising a switch control module 14, a GSR (galvanic skin response) detection module 16, a pulse control module 18, and a user biometric measuring module 20.

The stimulator 10 further comprises a high voltage generator 22 and current limiter 24 (and related current sensor 26) to generate stimulation pulses for application via a switching matrix 28.

The stimulator 10 further comprises user biometric measuring sensors 30 to determine physiological parameters associated with the user, and which is typically connected to the user biometric measuring module 20.

The stimulator 10 comprises a GSR sensor 32, in communication with the GSR detection module 16, to measure the electrical conductance of the user's skin.

The stimulator 10 comprises an applicator 34, which provides a contact surface for contacting the outer skin surface of the user, comprising a stimulus interface, typically in the form of an electrode pair 36. Clearly, if any of the other stimuli mentioned above, namely light, sound, magnetic field, vibration or pressure, are used, the stimulus interface would differ accordingly.

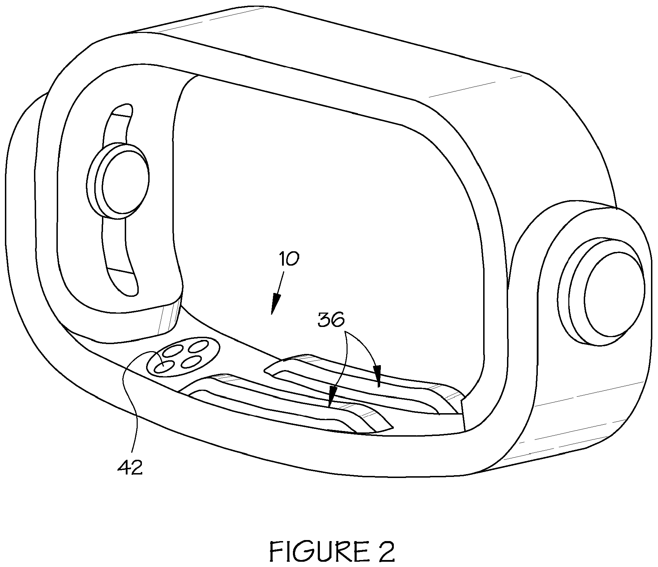

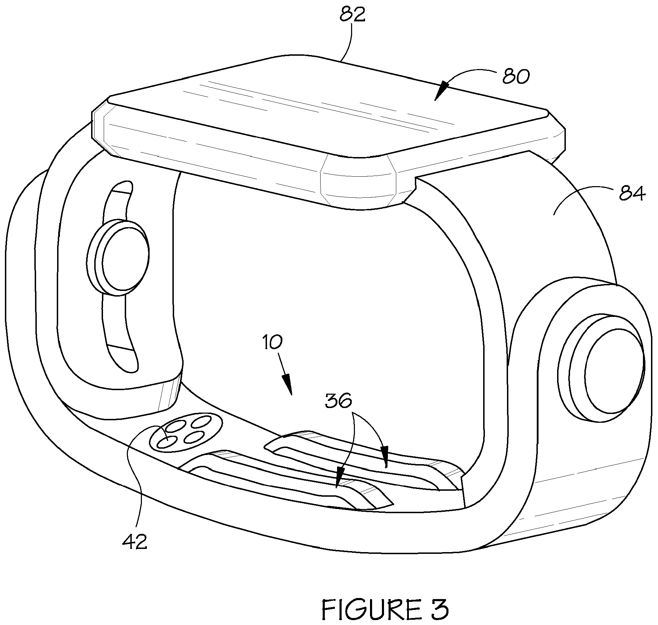

The electrode pair 36, applies the stimulation pulses to the user's skin, typically proximate the ventral side of the user's wrist 38 (as best shown in Figures 2 and 3, and which will be described in more detail further below). Although the electrodes of the electrode pair 36 are shown adjacent each other, in one version, only one electrode (e.g. cathode) may be secured to the user's arm, wrist or hand, with the other electrode (e.g. anode) being located on another part of the user's body.

Each electrode 36 has a related switching arrangement 40 connected to the switching matrix 28 to control the operation of the electrode 36.

The applicator 10 further comprises an optic sensor 42 to assist in determining the physiological parameters associated with the user, including heart rate (HR) (using light pulse diodes, for example), heart rate variability (HRV), temperature and SPO2 (to provide an estimate of the user's arterial oxygen saturation).

In an embodiment, a rechargeable battery 44 and related battery charger 46 are connected to the applicator 34, and in particular to each switching arrangement 40 associated with each electrode 36. A design aim of the nerve stimulator 10 of the present invention is to provide a stimulator with sufficiently few components/parameters to enable the stimulator 10 to take the form of a stand-alone device that is powered by the battery 44, which may take the form of a replaceable battery.

In an embodiment, each electrode 36 is a dual function electrode 36 that may provide and receive electrical current. The electrode pair 36, in addition to receiving electrical current to facilitate its operation, as described above, may be arranged to provide electrical charge to charge the rechargeable battery 44.

In an embodiment, the applicator 36 comprises a pair of electrodes to apply the stimulation pulses. In an embodiment, the electrodes 36 may be arranged into a predetermined configuration to allow targeted stimulation of specific areas of the nervous system. More specifically, a configuration of parallel electrodes may be applied to establish proximity for targeting the ulnar and the median nerves, which run alongside the length of the forearm (as shown in Figure 1). In one embodiment, two pairs of electrodes 36 may be provided in a criss-cross pattern to target the ulnar and median nerves.

In some embodiments, the sensor 42, for determining the physiological parameters associated with the user, and the electrodes 36 are spaced apart or segmented by at least 30 degrees around the circumference of the user's wrist 38, as shown in Figures 2 and 3.

Although not shown, the nerve stimulator may include an inertial measurement (IMU) module to determine whether the user is engaged in athletic activity.

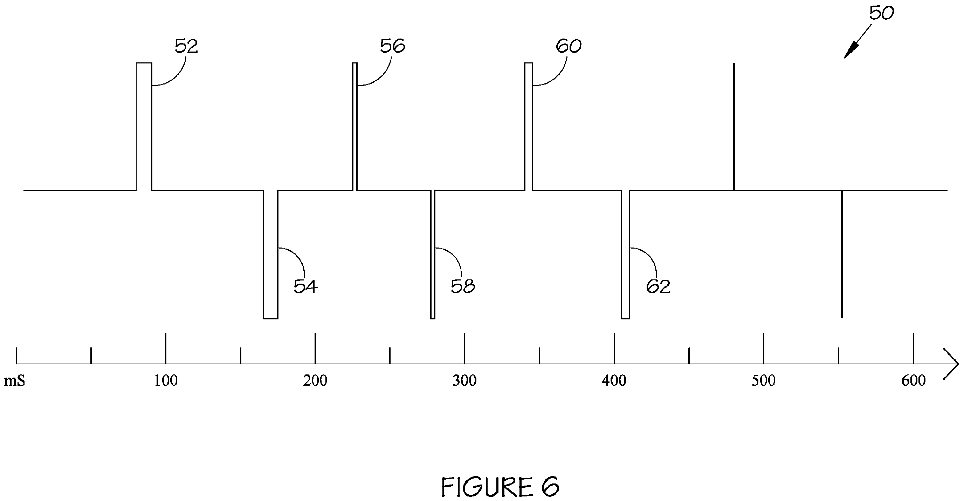

The pulse control module 18 is arranged to stimulate the nerves based on predetermined electrical stimulation patterns, comprising: stimulation session lengths, frequencies, amplitude and pulse width. An example of a stimulation waveform 50 of the type that may be generated by the nerve stimulator 10 of the invention is shown in Figure 6, comprising:
- Positive and negative pulses 52, 54 of a relatively long duration of 15 ms, spaced 90 ms apart;
- Positive and negative pulses 56, 58 of a relatively short duration of 5 ms, spaced 60 ms apart;
- Positive and negative pulses 60, 62 of a relatively intermediate duration of 10 ms, spaced 70 ms apart; and
- Positive and negative pulse bursts 64, 66, spaced 80 ms apart.;

Clearly, many other stimulation profiles may be used. In particular, a predetermined profile electrical stimulation may be used to affect certain conditions. In addition, different signal profiles may be useful for affecting different levels of the same condition (mild sleep problems vs. severe insomnia).

It is envisaged that superimposing distinct signals that have uniquely associated parameter profiles may have benefits for affecting a single or variety of conditions. In some embodiments, 3 or more signals may be superimposed upon one another. In yet other embodiments, superimposition may occur between signals of differing stimulation types for example when ultrasound simulation is coupled with non-invasive electrical stimulation. In one version, the pulse stimulation profile may have the following parameters:

| | |
|---|---|
| Frequency range: | 0.1 Hz to 1000 Hz |
| Intensity: | 100 uA to 10 mA |
| Pulsewidth: | 1 uS to 100 mS |

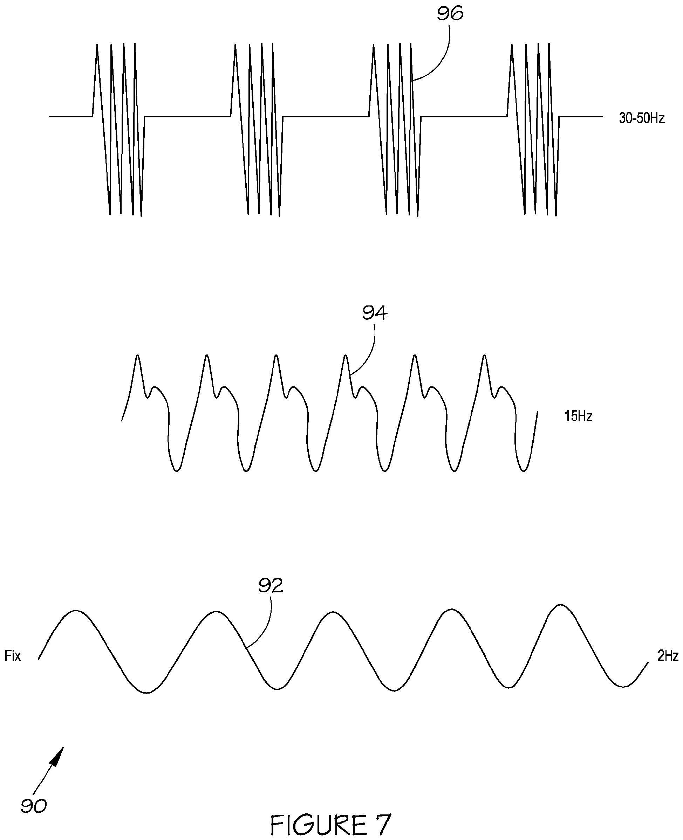

In a particular embodiment, turning now to Figure 7, the pulse stimulation profile 90 may comprise a slow oscillating pulse 92, having a frequency of around 1 Hz, a medium oscillating pulse 94 superimposed on top of the slow oscillating pulse having a frequency of around 15 Hz, and a high oscillating pulse 96 superimposed on top of the slow oscillating pulse and the medium oscillating pulse, having a frequency of between 30 Hz and 50 Hz.

Turning back to Figure 5, the nerve stimulator 10 may include a communication module 48 to facilitate communication and data transfer between the nerve stimulator 10 and an external/remote device, including the user's mobile device (i.e. smartphone, tablet or smartwatch) and/or a remote server on the cloud, the communication module 48 including a suitable communication component such as BlueTooth, RFID, NFC, Wi-Fi, ZigBee etc.

Conveniently, the nerve stimulator 10 may take the form of a portable, wearable stimulator, which may be embodied and/or housed and/or accommodated and/or integrated within and/or secured to (with a clip, for example) a band (as shown in Figure 2), smartwatch (as shown in Figure 3), patch, strap, or sleeve.

With reference to Figure 3 in particular, the nerve stimulator 10 may take the form of a portable, wearable stimulator, which may be coupled to a smartwatch 80 to form an integral device. A smartwatch 80 is a computerized wristwatch with extended functionality beyond timekeeping. Modern smartwatches 80 are effectively wearable computers, and as such have significantly expanded functionalities; many run mobile applications using a mobile operating system and in many cases emulate modern mobile telephones/smartphones.

In one particular application, the smartwatch 80 comprises a watch computing device 82 fitted to a contiguous strap 84, typically positioned on the dorsal side of the user's wrist, with the ventral side of the strap 84 comprising the nerve stimulator 10 of the present invention. Although they are separate and distinct components, the communication module 48 of the nerve stimulator 10 enables communications and/or the transfer of data and information between the watch computing device 82 and the nerve stimulator 10, typically wirelessly. In this application, the watch computing device 82 and the nerve stimulator 10 may be arranged diametrically opposite each other on the strap 84. The independent and separate controllers found in the watch computing device 82 and the nerve stimulator 10 allow for efficient coupling between two distinct electronics adorned to a user's wrist.

The communication module 48 of the nerve stimulator may be any suitable communication component (such as for BlueTooth, RFID, NFC, Wi-Fi, ZigBee etc.) for both transmitting stimulation data to and receiving biometric data from the processor located on the dorsal side of the wrist. In addition, the pulse control module 18 may also include controller logic to trigger electrical stimulation pulse profiles based on received biometric data from the smartwatch 80.

In an embodiment, the pulse control module 18 may also include controller logic to trigger electrical stimulation pulse profiles based on the physiological parameters received by the biometric measuring module 20 from the user biometric measuring sensors 30 and/or an indication from the IMU module regarding the user's physical activity, so as to define a closed loop bio-feedback arrangement. This leverages individual responses to stimulation, such as heart rate (HR) or heart rate variability (HRV) responses in the context of a fitness stimulation application, for example. In one particular application, although not limited thereto, this embodiment relates to the management of the user's heart rate, using stimulation of the median and/or ulnar nerves in a closed loop manner to achieve a desired physiological state.

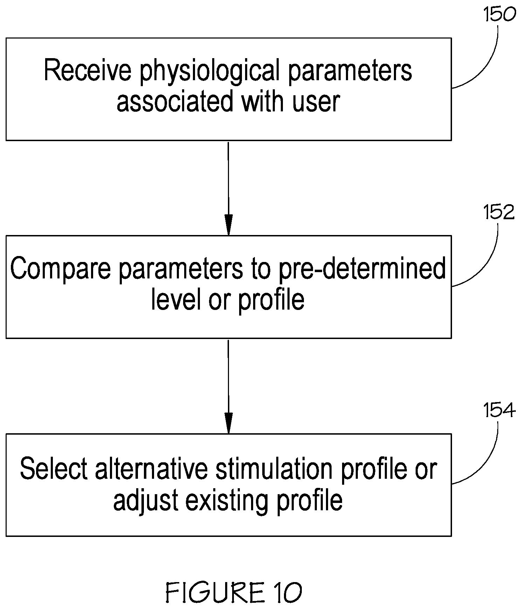

In one version, as shown in Figure 10, the pulse control module 18 may compare the received physiological parameters to a predetermined level or profile (blocks 150 and 152), and select an alternative electrical stimulation pulse profile (or adjust the existing profile being used), as shown in block 154. The pulse control module 18 continues to monitor the physiological parameters, and once the predetermined level or profile of the physiological parameters is achieved, yet another electrical stimulation pulse profile may be selected and applied to maintain the predetermined level or profile of the physiological parameters (or the stimulation may be stopped).

In this embodiment, the nerve stimulator may take the form of a wearable band, patch, or strap, of the type described above, which may be worn on the user's wrist, as shown in Figure 2.

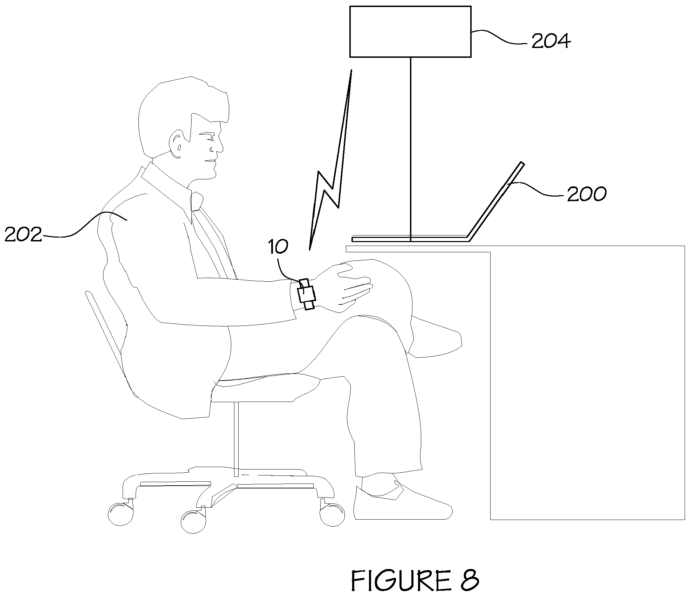

In an inventive embodiment, turning now to Figure 8, the nerve stimulator 10 may be used in conjunction with an interactive computer cognitive training application, such as a language or education software running on a training computer 200, which the user 202 may interact with. In this application, a training processing module 204 is provided to coordinate the application of the stimulation pulses with particular activities or learning elements that the user must engage with, and/or at predetermined times during the training. The aim of this particular embodiment is to coordinate learning, training and educating, either language or cognitive skills, with non-therapeutic stimulation of the median and/or ulnar nerves.

In one version, the training processing module 204 may monitor the user's performance during the training, and instruct the pulse control module 18 to select an electrical stimulation pulse profile (or select another electrical stimulation pulse profile, if one is already being used). The training processing module 204 continues to monitor the user's performance, and once a satisfactory performance level has been achieved, yet another electrical stimulation pulse profile may be selected and applied to maintain the user's performance (or the stimulation may be stopped).

Thus, in use, the training processing module 204 may identify previously failed attempts at questions or levels presenting difficulty to the user 202, and apply an appropriate amount of stimulation leading up to or at the time of the user 202 facing the challenging questions or levels.

In this embodiment, the nerve stimulator 10 may take the form of a wearable band, patch, or strap, of the type described above, which may be worn on the user's arm, wrist or hand, the nerve stimulator 10 being connected to (or at least in communication with) the training processing module 204 (via the communication module 48 and related communication component), which in turn is connected to (or at least in communication with) the training computer 200.

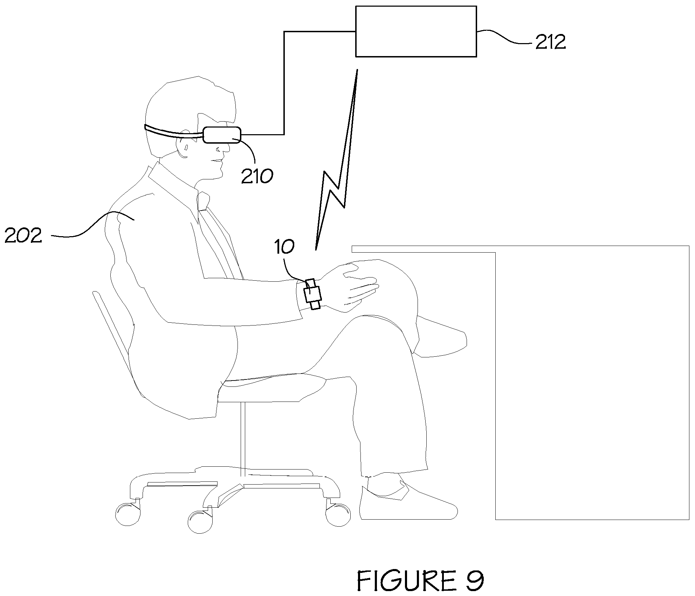

Turning now to Figure 9, in another embodiment, the nerve stimulator 10 may be used in conjunction with a visualizing device 210 running on a visualization computer, which the user may interact with to view (in the context of a virtual reality experience) corresponding anatomical movements during stimulation of the user's median and/or ulnar nerves. It is believed that stimulating the median nerve in tandem with visual perception of associated muscle movements will trigger pathways in the brain and regenerate lost motor pathways along the nervous system chain that connect the motor cortex through the central nervous system into the median and/or ulnar systems of the peripheral nervous system.

In this application, a visualization module 212 is provided to coordinate, typically in tandem, the application of the stimulation pulses to the median and/or ulnar nerves with the display on the visualizing device 210 of the movement of a muscle or muscle group associated with the median and/ulnar nerve. The visualizing device 210 may take the form of a virtual reality or ocular feedback device.

For example, in one version, the visualization module 212 may coordinate a virtually depicted movement or activity of a user's hand with electrical stimulation of associated anatomical locations. In an embodiment, the extent and nature of the virtually depicted activity or movement will coincide with the amount of stimulation provided.

In this embodiment, the nerve stimulator 10 may take the form of a wearable band, patch, or strap, of the type described above, which may be worn on the user's arm, wrist or hand, the nerve stimulator 10 being connected to (or at least in communication with) the visualization module 210 (via the communication module 48 and related communication component), which may in turn be connected to (or at least in communication with) the visualization computer.

The nerve stimulator 10 of the present invention aims to reduce the number of components/parameters to yield a smaller stimulation module that can be incorporated into a band or wristwatch, preferably to be situated on the dorsal side of the arm, wrist or hand. Such a device, in one embodiment, can send and receive signals with a smartwatch to which it is appended (or another local device such as a tablet or smart phone via Bluetooth) and may be internet connected for direct transceiving of data with a remote server via the cloud. In one embodiment, the stimulation module is embedded within the band clasp that makes contact with the wrist underside.

Over and above the applications described above, the nerve stimulator may be arranged to act as an alarm or notification system. For example, the user may set a wake-up time, typically via a device that is wirelessly connected to the stimulator. At the desired wake-up time, the nerve stimulator may impart a gradual stimulation to activate the user's nervous system and thus awaken the user. The alarm may also provide a notification means, such that the user is alerted when detected biometrics reach certain levels e.g., notify the user has low oxygen (from pulse oximeter), low/high heart rate (from a heart rate monitor), etc. In some embodiments, the alarm system may be tied to GPS/ proximity centre such that the user is alerted when he/she has gone off course from their intended destination or location.

Nerve stimulation can facilitate and optimize performance by direct sensory modulation of the sensory motor cortex, indirectly it might have an effect on heart rate variability (HRV) by modulating autonomic functioning and promoting a balance between the sympathetic and parasympathetic systems. By receiving sensory input from the median nerve at the same time a individual is performing a particular form of physical activity, the primary sensory cortex (S1) and the primary motor cortex (M1) will be engaged in the processing of such information through co-activation, this might facilitate plasticity at central level, but it can also improve motor output so the physical activity being performed benefits as the cortex is being primed by the combination of sensory processing. The effects of stimulating the median nerve and/or the ulnar nerve at S1 may also have an effect on pain processing, during exercise and as a result of different factors, pain can be a consequence of prolonged endurance, the modulation from thalamo-cortical circuits due to the stimulation, might increase pain threshold. Depending on the frequencies used, the endogenous opiod system can be stimulated to release enkephalins and endorphins at central and spinal level, thus helping to ease pain.

A specific set of parameters can be used with stochastic properties, where the frequencies generated ranges from 0.5 to 100 Hz and not limited to higher ranges (no more 1000 Hz). The current is delivered in a range of 1 to 6 mA fluctuating over time and in a series of mixed pulses variable characteristics (quadratic, triangular or sinusoidal). Stimulation of the median nerve and/or the ulnar nerve can be coupled with monitoring functions such as heart rate (HR), pulse, and HRV. It can also be used in combination with a system providing biofeedback for heart and breathing capabilities, thus, if integrated stimulation of the median nerve and/or the ulnar nerve can be included in a closed -loop system for stimulation and physiological entrainment. Because the activation of S1 and its close relationship with the fronto-temporal network, attention can be enhanced by stimulation of the median nerve and/or the ulnar nerve, as in the case for HR or HRV, monitoring of cognitive performance through cognitive testing is feasible as well. Sustained attention in specific cognitive tasks will be the fundament for the development of a closed-loop system for cognitive performance. This makes stimulation of the median nerve and/or the ulnar nerve suitable for cognitive and behavioral interventions aimed to optimize learning, reading, attention or multitasking.

In addition, this disclosure suggests a co-activation process. In particular, the neural level, can be understood as a way to engage a particular neural system that is already being active, in the processing of another type of information coming from another neural unit, these units interact creating thus a network that the two systems form. This shared and simultaneous activation can boost the processing of the entire network. Behaviorally, this can be applied to facilitate the effects of one technique or method by improving the integration and processing of such information.

## Claims

1. A system comprising:
a non-invasive nerve stimulator (10, 100) for peripheral nerves, including the ulnar and/or median nerves, configured to be fitted proximate the left and/or right arm, wrist (38) or hand of a user (202), to stimulate the median and/or ulnar nerves using at least one stimulus, the nerve stimulator (10, 100) comprising:
a stimulus generator (102) to generate at least one stimulus;
an applicator (34, 104) to apply the generated stimulus to the arm, wrist (38) or hand of the user (202), the applicator (34, 104) comprising at least one stimulus interface to apply the stimulus to the user's skin, proximate to the anterior or ventral side of the user's arm, wrist (38) or hand; and
at least one user biometric measuring sensor (30, 108), embodied in or proximate the applicator (34, 104), to monitor and determine physiological parameters associated with the user (202), including any one or more of the following: heart rate, HR, heart rate variability, HRV, temperature, SPO2, galvanic skin response, GSR, and inertial measurement;
a controller (12, 106) to control the operation of the stimulus generator (102) and the applicator (34, 104), the controller (12, 106) including a stimulus controller module (112) to control the applied stimulation in accordance with a stimulation profile, the stimulation profile defining the applied stimulus in terms of duration and/or frequency and/or intensity/amplitude and/or width, wherein the stimulus controller module applies a stimulation profile based on the physiological parameters received from the at least one user biometric measuring sensor (30, 108), so as to define a closed-loop feedback arrangement, wherein the stimulus controller module (112) compares the received physiological parameters to a predetermined level or profile, and either selects an alternative stimulation profile or adjusts the existing stimulation profile being used,
**characterised in that** the system includes:
a training computer (200) running an interactive computer cognitive training application, which the user (202) is arranged to interact with, the training computer (200) comprising a training processing module (204), which is arranged to be in communication with the nerve stimulator (10, 100), to coordinate the stimulation of the median and/or ulnar nerves with particular activities or learning elements that the user (202) must engage with, and/or at predetermined times during the training.

2. The system of claim 1, wherein the stimulus controller module (112) is configured to continue to monitor the physiological parameters, and once the predetermined level or profile of the physiological parameters is achieved, yet another stimulation profile may be selected and applied by the stimulus controller module (112) to maintain the predetermined level or profile of the physiological parameters, or the stimulation may be stopped.

3. The system of either claim 1 or claim 2, wherein the controller (12, 106) includes:
a GSR (galvanic skin response) detection module (16) in communication with a GSR sensor (32), to measure the electrical conductance of the user's skin; and
an inertial measurement, IMU, module in communication with a IMU sensor to determine whether the user (202) is engaged in athletic activity, further wherein the user biometric measuring sensors (30, 108), for monitoring and determining the physiological parameters associated with the user (202), and the stimulus interface are spaced apart or segmented by at least 30 degrees around the circumference of the user's arm, wrist (38) or hand.

4. The system of any one of the preceding claims, wherein the stimulator (10, 100) includes a communication module (114) to facilitate communication and data transfer between the nerve stimulator (10, 100) and an external or remote device, including the user's mobile device and/or a remote server, wherein the data transferred from the stimulator (10, 100) includes information regarding the stimulus being applied, including duration, frequency and strength and/or the physiological parameters determined by the biometric measuring sensors (30, 108) and wherein the data transferred to the stimulator (10, 100) from the external or remote device includes information regarding a variation to the stimulus to be applied, to implement an amended or adjusted stimulation profile, which the controller (12, 106) can then implement, via the stimulus controller module (112), in conjunction with the generator (102) and applicator (34, 104).

5. The system of claim 4, wherein the stimulator (10, 100) or a plurality of stimulators (10, 100) is/are fitted so as to stimulate the user's left arm and/or wrist (38) and/or hand, the user's right arm and/or wrist (38) and/or hand, or the user's left and right arm and/or wrist (38) and/or hand further wherein the at least one stimulus includes electricity, light, sound, magnetic field, vibration or pressure, or any combination of these stimuli, with the stimulus being applied either as a continuous waveform, including square, rectangular, sinusoidal or triangular waveforms, or as a series of pulses wherein:
for a magnetic stimulus, the stimulus generator (102) includes a time varying magnetic field generator and related stimulus interface to induce an electrical current in the user's tissue, in order to stimulate the nerve/s;
for light stimulus, the stimulus generator (102) includes a light source, selected from a group comprising a light-emitting diode (LED) and a laser, in order to stimulate the nerve/s;
for a sound stimulus, the stimulus generator (102) includes a sound generator in order to stimulate the nerve/s;
for a vibration stimulus, the stimulus generator (102) includes a vibration generator, in order to stimulate the nerve/s; and
for a pressure stimulus, the stimulus generator (102) includes a vibration generator, in order to stimulate the nerve/s.

6. The system of claim 5, wherein for an electrical stimulus, the stimulus generator (102) includes a high voltage generator (22) and a current limiter (24) to generate the electrical stimulus for application via a switching matrix (28), wherein the electrical stimulus comprises a series of electrical pulses, with the stimulus controller module (112) including a switch control module (14) and a pulse control module (18) to control switching of the electrical stimulus, via the switching matrix (28), in accordance with the stimulation profile, and wherein the stimulus interface includes at least one switching arrangement (40) connected to the switching matrix (28) to control the operation of the stimulus interface, further wherein a battery (44) and related battery charger (46) are connected to each switching arrangement (40) associated with each stimulus interface, wherein the stimulus interface includes at least one electrode pair (36) to send current through the user's tissue in order to stimulate the relevant nerve/s, and wherein at least one electrode pair (36) has a dual function to enable the electrode pair (36) to provide and receive electrical current with the electrode pairs (36) being arranged into a predetermined configuration to allow targeted stimulation of specific areas of the nervous system, with the electrode pair (36) either running parallel to each other for targeting the ulnar and/or the median nerves, which run alongside the length of the forearm, or being provided in a criss-cross pattern to target the ulnar and/or median nerves.

7. The system of claim 6, wherein in the case of a series of electrical pulses, each stimulation pulse comprises a pulse of a single frequency or a combination of two or more pulses of the same or different frequencies, wherein the pulse stimulation profile is in the frequency range of between 0.1 Hz to 1000 Hz, has an intensity of between 100 uA to 10 mA and a pulse width of between 1 uS to 100 mS, wherein the stimulation comprises the sum of two or more frequencies, including a slow oscillating waveform, having a frequency of around 1 Hz, a medium oscillating waveform superimposed on top of the slow oscillating waveform having a frequency of around 15 Hz, and a high oscillating waveform superimposed on top of the slow waveform pulse and the medium oscillating waveform, having a frequency of between 30 Hz and 50 Hz.

8. The system of either claims 6 or 7, wherein the training processing module (204) monitors the user's performance during the training, and instructs the pulse control module (18) to select an electrical stimulation pulse profile or select another electrical stimulation pulse profile, if one is already being used.

9. The system of claim 8, wherein the training processing module (204) continues to monitor the user's performance, and once a required performance level has been achieved, another electrical stimulation pulse profile is selected and applied to maintain the user's performance.

10. The system of either claim 8 or claim 9, wherein the nerve stimulator (10, 100) is in communication with the training processing module (204) via the communication module (48, 114), which in turn is in communication with the training computer (200).

11. The system of any one of the preceding claims, wherein the nerve stimulator (10, 100) takes the form of a portable, wearable stimulator (10, 100), embodied and/or housed and/or accommodated and/or integrated within and/or secured to a wearable device, including a fitness tracker band, clasp, patch, strap (84), or sleeve or watch or smartwatch (80), wherein the watch makes use of a clasp arrangement comprising a central, curved clipping body, to which adjacent flexible strap portions are hingedly fitted, with the nerve stimulator (10, 100) being integrally fitted, or removably fittable, to the clipping body.

12. The system of claim 11, wherein in the case of a smartwatch (80), the smartwatch (80) comprises a watch device fitted to a strap (84), positioned on the dorsal side of a user's wrist (38) in use, the watch device including a computing device (82).

13. A non-therapeutic method of operating a system comprising a training computer (200) and a non-invasive nerve stimulator (10, 100) for peripheral nerves, including the ulnar and/or median nerves, to stimulate the median and/or ulnar nerves using at least one stimulus, the nerve stimulator (10, 100) comprising a stimulus generator (102), to generate at least one stimulus according to a stimulation profile, an applicator (34, 104) to apply the generated stimulus to the arm, wrist (38) or hand of the user (202), and a controller (12, 106) to control the operation of the stimulus generator (102) and the applicator (34, 104), the method comprising
receiving at least one physiological parameter associated with a user (202);
comparing the at least one received physiological parameter to a predetermined level or profile (150, 152);
selecting an alternative stimulation profile, or adjust the existing stimulation profile being used, accordingly (154); and
**characterised in that** the non-therapeutic method further comprises:
running an interactive computer cognitive training application on the training computer (200), which the user (202) is arranged to interact with, the training computer (200) being in communication with the nerve stimulator (10, 100);
monitoring the user's interaction with the interactive computer cognitive training application on the training computer (200); and
coordinating the application of the stimulus with particular activities or learning elements that the user (202) must engage with and/or at predetermined times during the training.

14. The non-therapeutic method of claim 13, wherein the non-therapeutic method further includes the step of continuing to monitor the at least one physiological parameter, and once the predetermined level or profile of the physiological parameters is achieved, the method includes selecting another stimulation profile to maintain the predetermined level or profile of the at least one physiological parameter, or stopping the stimulation.

15. The non-therapeutic method of either claim 13 or 14, wherein the non-therapeutic method further includes the steps of:
monitoring the user's performance during the training, and instructing the stimulus controller module (112) to select a stimulation profile or to select another stimulation profile, if one is already being used;
continuing to monitor the user's performance, and once a predetermined performance level has been achieved, selecting another stimulation profile or stopping the stimulation.

## Patentansprüche

1. System umfassend:
einen nicht-invasiven Nervenstimulator (10, 100) für periphere Nerven, einschließlich der Ellennerven (Nervus ulnaris) und/oder Mittelarmnerven (Nervus medianus), der so konfiguriert ist, dass er in der Nähe des linken und/oder rechten Arms, Handgelenks (38) oder der Hand eines Benutzers (202) angebracht werden kann, um die Mittelarmnerven und/oder Ellennerven unter Verwendung mindestens eines Stimulus zu stimulieren, wobei der Nervenstimulator (10, 100) umfasst:
einen Stimulusgenerator (102) zur Erzeugung mindestens eines Stimulus;
einen Applikator (34, 104), um den erzeugten Stimulus auf den Arm, das Handgelenk (38) oder die Hand des Benutzers (202) anzuwenden, wobei der Applikator (34, 104) mindestens eine Stimulusschnittstelle umfasst, um den Stimulus auf die Haut des Benutzers in der Nähe der vorderen oder ventralen Seite des Arms, des Handgelenks (38) oder der Hand des Benutzers anzuwenden; und
mindestens einen biometrischen Messsensor (30, 108) für den Benutzer, der in oder in der Nähe des Applikators (34, 104) angeordnet ist, um physiologische Parameter, die mit dem Benutzer (202) verbunden sind, zu überwachen und zu bestimmen, einschließlich einen oder mehrere der folgenden Parameter: Herzfrequenz, HR, Herzfrequenzvariabilität, HRV, Temperatur, SPO2, galvanische Hautreaktion, GSR, und Trägheitsmessung;
ein Steuergerät (12, 106) zur den Betrieb des Stimulusgenerators (102) und des Applikators (34, 104) zu steuern, wobei das Steuergerät (12, 106) ein Stimulussteuermodul (112) umfasst, um die angewandte Stimulation in Übereinstimmung mit einem Stimulationsprofil zu steuern, wobei das Stimulationsprofil den angewandten Stimulus in Bezug auf Dauer und/oder Frequenz und/oder Intensität/Amplitude und/oder Breite definiert, wobei das Stimulussteuermodul ein Stimulationsprofil anwendet, das auf den physiologischen Parametern basiert, die von dem mindestens einen biometrischen Messsensor (30, 108) des Benutzers empfangen werden, um eine Rückkopplungsanordnung mit geschlossenem Regelkreis zu definieren, wobei das Stimulussteuermodul (112) die empfangenen physiologischen Parameter mit einem vorbestimmten Niveau oder Profil vergleicht und entweder ein alternatives Stimulationsprofil auswählt oder das bestehende, verwendete Stimulationsprofil anpasst,
**dadurch gekennzeichnet, dass** das System umfasst:
einen Trainingscomputer (200), auf dem eine interaktive kognitive Computer-Trainingsanwendung läuft, mit der der Benutzer (202) interagieren kann, wobei der Trainingscomputer (200) ein Trainingsverarbeitungsmodul (204) umfasst, das so angeordnet ist, dass es mit dem Nervenstimulator (10, 100) in Verbindung steht, um die Stimulation der Mittelarmnerven und/oder Ellennerven mit bestimmten Aktivitäten oder Lernelementen zu koordinieren, mit denen sich der Benutzer (202) beschäftigen muss, und/oder zu vorbestimmten Zeiten während des Trainings.

2. System nach Anspruch 1, wobei das Stimulussteuermodul (112) so konfiguriert ist, dass es die Überwachung der physiologischen Parameter fortsetzt, und sobald das vorbestimmte Niveau oder Profil der physiologischen Parameter erreicht ist, ein weiteres Stimulationsprofil ausgewählt und durch das Stimulussteuermodul (112) angewendet werden kann, um das vorbestimmte Niveau oder Profil der physiologischen Parameter beizubehalten, oder die Stimulation gestoppt werden kann.

3. System nach Anspruch 1 oder Anspruch 2, wobei das Steuergerät (12, 106) umfasst:
ein GSR-Erfassungsmodul (16) (galvanische Hautreaktion) das mit einem GSR-Sensor (32) in Verbindung steht, um die elektrische Leitfähigkeit der Haut des Benutzers zu messen; und
ein Trägheitsmessmodul (IMU), das mit einem IMU-Sensor in Verbindung steht, um festzustellen, ob der Benutzer (202) eine sportliche Aktivität ausübt, wobei ferner die biometrischen Messsensoren (30, 108) des Benutzers zum Überwachen und Bestimmen der physiologischen Parameter, die dem Benutzer (202) zugeordnet sind, und die Stimulusschnittstelle um mindestens 30 Grad um den Umfang des Arms, des Handgelenks (38) oder der Hand des Benutzers voneinander beabstandet oder getrennt sind.

4. System nach einem der vorhergehenden Ansprüche, wobei der Stimulator (10, 100) ein Kommunikationsmodul (114) umfasst, um die Kommunikation und den Datentransfer zwischen dem Nervenstimulator (10, 100) und einem externen oder entfernten Gerät, einschließlich des mobilen Geräts des Benutzers und/oder eines entfernten Servers, zu erleichtern, wobei die von dem Stimulator (10, 100) übertragenen Daten Informationen über den angewandten Stimulus, einschließlich Dauer, Frequenz und Stärke, und/oder der physiologischen Parameter, die von den biometrischen Messsensoren (30, 108) bestimmt werden, enthalten, und wobei die von der externen oder entfernten Vorrichtung an den Stimulator (10, 100) übertragenen Daten Informationen über eine Variation des anzuwendenden Stimulus enthalten, um ein geändertes oder angepasstes Stimulationsprofil zu implementieren, das das Steuergerät (12, 106) dann über das Stimulussteuermodul (112) in Verbindung mit dem Generator (102) und dem Applikator (34, 104) implementieren kann.

5. System nach Anspruch 4, wobei der Stimulator (10, 100) oder mehrere Stimulatoren (10, 100) so angebracht ist/sind, dass er/sie den linken Arm und/oder das Handgelenk (38) und/oder die Hand des Benutzers, den rechten Arm und/oder das Handgelenk (38) und/oder die Hand des Benutzers oder den linken und rechten Arm und/oder das Handgelenk (38) und/oder die Hand des Benutzers stimuliert/stimulieren, wobei der mindestens eine Stimulus Elektrizität, Licht, Schall, ein Magnetfeld, Vibration oder Druck oder eine beliebige Kombination dieser Stimuli umfasst, wobei der Stimulus entweder als eine kontinuierliche Wellenform, einschließlich quadratischer, rechteckiger, sinusförmiger oder dreieckiger Wellenformen, oder als eine Reihe von Impulsen angelegt wird, wobei:
der Stimulusgenerator (102) für einen magnetischen Stimulus, einen zeitlich variierenden Magnetfeldgenerator und eine entsprechende Stimulusschnittstelle umfasst, um einen elektrischen Strom im Gewebe des Benutzers zu induzieren, um den/die Nerv/en zu stimulieren;
der Stimulusgenerator (102) für die Lichtstimulation eine Lichtquelle umfasst, die aus einer Gruppe ausgewählt wird, die eine Leuchtdiode (LED) und einen Laser umfasst, um den/die Nerv/en zu stimulieren;
der Stimulusgenerator (102) für einen akustischen Stimulus einen Tongenerator umfasst, um den/die Nerv/en zu stimulieren;
der Stimulusgenerator (102) für einen Vibrationsstimulus einen Vibrationsgenerator umfasst, um den/die Nerv/en zu stimulieren; und
der Stimulusgenerator (102) für einen Druckstimulus einen Vibrationsgenerator umfasst, um den/die Nerv/en zu stimulieren.

6. System nach Anspruch 5, wobei der Stimulusgenerator (102) für einen elektrischen Stimulus einen Hochspannungsgenerator (22) und einen Strombegrenzer (24) umfasst, um den elektrischen Stimulus zur Anwendung über eine Schaltmatrix (28) zu erzeugen, wobei der elektrische Stimulus eine Reihe von elektrischen Impulsen umfasst, wobei das Stimulussteuermodul (112) ein Schaltsteuermodul (14) und ein Impulssteuermodul (18) umfasst, um das Schalten des elektrischen Stimulus über die Schaltmatrix (28) in Übereinstimmung mit dem Stimulationsprofil zu steuern, und wobei die Stimulusschnittstelle mindestens eine Schaltanordnung (40) umfasst, die mit der Schaltmatrix (28) verbunden ist, um den Betrieb der Stimulusschnittstelle zu steuern, wobei ferner eine Batterie (44) und ein zugehöriges Batterieladegerät (46) mit jeder Schaltanordnung (40) verbunden sind, die mit jeder Stimulusschnittstelle verbunden ist, wobei die Stimulusschnittstelle mindestens ein Elektrodenpaar (36) umfasst, um Strom durch das Gewebe des Benutzers zu senden, um den/die relevanten Nerv/en zu stimulieren, und wobei mindestens ein Elektrodenpaar (36) eine Doppelfunktion hat, um das Elektrodenpaar (36) in die Lage zu versetzen, elektrischen Strom zu liefern und zu empfangen, wobei die Elektrodenpaare (36) in einer vorbestimmten Konfiguration angeordnet sind, um eine gezielte Stimulation spezifischer Bereiche des Nervensystems zu ermöglichen, wobei das Elektrodenpaar (36) entweder parallel zueinander verläuft, um auf die Mittelarmnerven und/oder Ellennerven abzuzielen, die entlang der Länge des Unterarms verlaufen, oder in einem kreuz und quer verlaufenden Muster vorgesehen ist, um auf die Ellennerven und/oder Mittelarmnerven abzuzielen.

7. System nach Anspruch 6, wobei im Falle einer Reihe von elektrischen Impulsen jeder Stimulationsimpuls einen Impuls mit einer einzigen Frequenz oder eine Kombination von zwei oder mehr Impulsen mit denselben oder unterschiedlichen Frequenzen umfasst, wobei das Impulsstimulationsprofil im Frequenzbereich zwischen 0.1 Hz bis 1000 Hz liegt, eine Intensität zwischen 100 uA und 10 mA und eine Impulsbreite zwischen 1 uS und 100 mS aufweist, wobei die Stimulation die Summe von zwei oder mehr Frequenzen umfasst, einschließlich einer langsamen oszillierenden Wellenform mit einer Frequenz von etwa 1 Hz, einer mittleren oszillierenden Wellenform, die der langsamen oszillierenden Wellenform überlagert ist, mit einer Frequenz von etwa 15 Hz, und einer hohen oszillierenden Wellenform, die dem langsamen Wellenformimpuls und der mittleren oszillierenden Wellenform überlagert ist, mit einer Frequenz zwischen 30 Hz und 50 Hz.

8. System nach einem der Ansprüche 6 oder 7, wobei das Trainingsverarbeitungsmodul (204) die Leistung des Benutzers während des Trainings überwacht und das Impulssteuerungsmodul (18) anweist, ein elektrisches Stimulationsimpulsprofil auszuwählen oder ein alternatives elektrisches Stimulationsimpulsprofil auszuwählen, wenn bereits eines verwendet wird.

9. System nach Anspruch 8, wobei das Trainingsverarbeitungsmodul (204) die Leistung des Benutzers weiterhin überwacht und, sobald ein erforderliches Leistungsniveau erreicht ist, ein alternatives elektrisches Stimulationsimpulsprofil ausgewählt und angewendet wird, um die Leistung des Benutzers aufrechtzuerhalten.

10. System nach Anspruch 8 oder 9, wobei der Nervenstimulator (10, 100) über das Kommunikationsmodul (48, 114) mit dem Trainingsverarbeitungsmodul (204) kommuniziert, das wiederum mit dem Trainingscomputer (200) kommuniziert.

11. System nach einem der vorhergehenden Ansprüche, wobei der Nervenstimulator (10, 100) die Form eines transportablen, tragbaren Stimulators (10, 100) hat, der in einem tragbaren Gerät, einschließlich eines Fitness-Tracker-Bands, einer Spange, eines Pflasters, eines Armbands (84) oder einer Stulpe oder einer Uhr oder Smartwatch (80), verkörpert und/oder untergebracht und/oder verstaut und/oder integriert und/oder daran befestigt ist, wobei die Uhr eine Verschlussanordnung verwendet, die einen zentralen, gekrümmten Verbindungskörper umfasst, an dem benachbarte flexible Bandabschnitte gelenkig angebracht sind, wobei der Nervenstimulator (10, 100) integral an dem Verbindungskörper angebracht oder abnehmbar angebracht werden kann.

12. System nach Anspruch 11, wobei im Falle einer Smartwatch (80) die Smartwatch (80) eine Uhrvorrichtung umfasst, die an einem Armband (84) angebracht ist, das an der Rückenseite des Handgelenks (38) eines Benutzers (38) positioniert ist, wobei die Uhrvorrichtung eine Rechenvorrichtung (82) umfasst.

13. Nicht-therapeutisches Verfahren zum Betreiben eines Systems, das einen Trainingscomputer (200) und einen nicht-invasiven Nervenstimulator (10, 100) für periphere Nerven, einschließlich der Ellennerven (Nervus ulnaris) und/oder Mittelarmnerven (Nervus medianus), umfasst, um die Mittelarmnerven und/oder Ellennerven unter Verwendung mindestens eines Stimulus zu stimulieren, wobei der Nervenstimulator (10, 100) einen Stimulusgenerator (102) umfasst, um mindestens einen Stimulus gemäß einem Stimulationsprofil zu erzeugen, einen Applikator (34, 104), um den erzeugten Stimulus auf den Arm, das Handgelenk (38) oder die Hand des Benutzers (202) aufzubringen, und eine Steuerung (12, 106), um den Betrieb des Stimulusgenerators (102) und des Applikators (34, 104) zu steuern, wobei das Verfahren umfasst
Empfang mindestens einen physiologischer Parameter, der einem Benutzer zugeordnet ist (202);
Vergleich des mindestens einen empfangenen physiologischen Parameters mit einem vorgegebenen Wert oder Profil (150, 152);
Auswahl eines alternativen Stimulationsprofils oder entsprechende Anpassung des bereits verwendeten Stimulationsprofils (154); und
**dadurch gekennzeichnet, dass** das nicht-therapeutische Verfahren ferner umfasst:
Ausführen einer interaktiven kognitiven Computer-Trainingsanwendung auf dem Trainingscomputer (200), mit dem der Benutzer (202) interagieren kann, wobei der Trainingscomputer (200) mit dem Nervenstimulator (10, 100) in Verbindung steht;
Überwachung der Interaktion des Benutzers mit der interaktiven kognitiven Computer-Trainingsanwendung auf dem Trainingscomputer (200); und
Koordinierung der Anwendung des Stimulus mit bestimmten Aktivitäten oder Lernelementen, mit denen sich der Benutzer (202) beschäftigen muss, und/oder zu vorbestimmten Zeiten während des Trainings.

14. Nicht-therapeutisches Verfahren nach Anspruch 13, wobei das nicht-therapeutische Verfahren ferner den Schritt der fortgesetzten Überwachung des mindestens einen physiologischen Parameter zu umfasst, und sobald das vorbestimmte Niveau oder Profil der physiologischen Parameter erreicht ist, das Verfahren die Auswahl eines alternativen Stimulationsprofils, um das vorbestimmte Niveau oder Profil des mindestens eines physiologischen Parameters aufrechtzuerhalten, oder das Stoppen der Stimulation umfasst.

15. Das nicht-therapeutische Verfahren nach Anspruch 13 oder 14, wobei das nicht-therapeutische Verfahren ferner die folgenden Schritte umfasst:
Überwachung der Leistung des Benutzers während des Trainings und Anweisung an das Stimulussteuermodul (112), ein Stimulationsprofil auszuwählen oder ein alternatives Stimulationsprofil auszuwählen, wenn bereits eines verwendet wird;
Weitere Überwachung der Leistung des Benutzers und Auswahl eines alternativen Stimulationsprofil nach Erreichen eines bestimmten Leistungsniveaus oder Beenden der Stimulation.

## Revendications

1. Un système comprenant:
un stimulateur nerveux non invasif (10, 100) pour les nerfs périphériques, y compris les nerfs ulnaire et/ou médian, configuré pour être installé à proximité du bras gauche et/ou droit, du poignet (38) ou de la main d'un utilisateur (202), pour stimuler les nerfs médian et/ou ulnaire à l'aide d'au moins un stimulus, le stimulateur nerveux (10, 100) comprenant:
un générateur de stimulus (102) pour générer au moins un stimulus;
un applicateur (34, 104) pour appliquer le stimulus généré au bras, au poignet (38) ou à la main de l'utilisateur (202), l'applicateur (34, 104) comprenant au moins une interface de stimulus pour appliquer le stimulus à la peau de l'utilisateur, à proximité de la face antérieure ou ventrale du bras, du poignet (38) ou de la main de l'utilisateur; et
au moins un capteur de mesure biométrique pour l'utilisateur (30, 108), incorporé dans ou à proximité de l'applicateur (34, 104), pour surveiller et déterminer les paramètres physiologiques associés à l'utilisateur (202), y compris un ou plusieurs des éléments suivants fréquence cardiaque, FC, variabilité de la fréquence cardiaque, VFC, température, SPO2, réponse galvanique de la peau, RSG, et mesure inertielle;
un contrôleur (12, 106) pour contrôler le fonctionnement du générateur de stimulus (102) et de l'applicateur (34, 104), le contrôleur (12, 106) comprenant un module de contrôle de stimulus (112) pour contrôler la stimulation appliquée conformément à un profil de stimulation, le profil de stimulation définissant le stimulus appliqué en termes de durée et/ou de fréquence et/ou d'intensité/amplitude et/ou de largeur, le module de contrôle de stimulus applique un profil de stimulation basé sur les paramètres physiologiques reçus d'au moins un capteur de mesure biométrique de l'utilisateur (30, 108), de manière à définir un dispositif de rétroaction en boucle fermée, le module de contrôle de stimulus (112) comparant les paramètres physiologiques reçus à un niveau ou à un profil prédéterminé et sélectionnant un autre profil de stimulation ou ajustant le profil de stimulation existant en cours d'utilisation,
**caractérisé par le fait que** le système comprend
un ordinateur d'entraînement (200) exécutant une application informatique interactive de formation cognitive, avec laquelle l'utilisateur (202) est censé interagir, l'ordinateur d'entraînement (200) comprenant un module de traitement de l'entraînement (204), qui est censé être en communication avec le stimulateur nerveux (10, 100), pour coordonner la stimulation des nerfs médian et/ou ulnaire avec des activités particulières ou des éléments d'apprentissage auxquels l'utilisateur (202) doit participer, et/ou à des moments prédéterminés au cours de la formation.

2. Le système de la revendication 1, dans lequel le module de contrôle de stimulus (112) est configuré pour continuer à surveiller les paramètres physiologiques et, une fois que le niveau ou le profil prédéterminé des paramètres physiologiques est atteint, un autre profil de stimulation peut être sélectionné et appliqué par le module de contrôle de stimulus (112) pour maintenir le niveau ou le profil prédéterminé des paramètres physiologiques, ou la stimulation peut être interrompue.

3. Le système de la revendication 1 ou de la revendication 2, dans lequel le contrôleur (12, 106) comprend:
un module de détection GSR (réponse galvanique de la peau) (16) en communication avec un capteur GSR (32), pour mesurer la conductance électrique de la peau de l'utilisateur; et
un module de mesure inertielle (IMU) en communication avec un capteur IMU pour déterminer si l'utilisateur (202) est engagé dans une activité athlétique, dans lequel les capteurs de mesure biométrique de l'utilisateur (30, 108), pour surveiller et déterminer les paramètres physiologiques associés à l'utilisateur (202), et l'interface de stimulus sont espacés ou segmentés d'au moins 30 degrés autour de la circonférence du bras, du poignet (38) ou de la main de l'utilisateur.

4. Le système de l'une quelconque des revendications précédentes, dans lequel le stimulateur (10, 100) comprend un module de communication (114) pour faciliter la communication et le transfert de données entre le stimulateur nerveux (10, 100) et un dispositif externe ou distant, y compris le dispositif mobile de l'utilisateur et/ou un serveur distant, dans lequel les données transférées à partir du stimulateur (10, 100) comprennent des informations concernant le stimulus appliqué, y compris la durée, fréquence et force et/ou les paramètres physiologiques déterminés par les capteurs de mesure biométrique (30, 108) et dans lequel les données transférées au stimulateur (10, 100) à partir du dispositif externe ou distant comprennent des informations concernant une variation du stimulus à appliquer, pour mettre en oeuvre un profil de stimulation modifié ou ajusté, que le contrôleur (12, 106) peut ensuite mettre en oeuvre, via le module de contrôle de stimulus (112), en conjonction avec le générateur (102) et l'applicateur (34, 104).

5. Le système de la revendication 4, dans lequel le stimulateur (10, 100) ou une pluralité de stimulateurs (10, 100) est/sont installé(s) de manière à stimuler le bras gauche et/ou le poignet (38) et/ou la main de l'utilisateur, le bras droite et/ou le poignet (38) et/ou la main de l'utilisateur, ou le bras gauche et droite et/ou le poignet (38) et/ou la main de l'utilisateur, et dans lequel le stimulus au moins comprend de l'électricité, la lumière, le son, le champ magnétique, la vibration ou la pression, ou toute combinaison de ces stimuli, le stimulus étant appliqué soit sous la forme d'une onde continue, notamment carrée, rectangulaire, sinusoïdale ou triangulaire, soit sous la forme d'une série d'impulsions, dans laquelle:
pour un stimulus magnétique, le générateur de stimulus (102) comprend un générateur de champ magnétique variable dans le temps et une interface de stimulus connexe pour induire un courant électrique dans le tissu de l'utilisateur, afin de stimuler le(s) nerf(s);
pour le stimulus lumineux, le générateur de stimulus (102) comprend une source lumineuse, choisie dans un groupe comprenant une diode électroluminescente (DEL) et un laser, afin de stimuler le(s) nerf(s);
pour un stimulus sonore, le générateur de stimulus (102) comprend un générateur de son afin de stimuler le(s) nerf(s);
pour un stimulus vibratoire, le générateur de stimulus (102) comprend un générateur de vibrations, afin de stimuler le(s) nerf(s); et
pour un stimulus de pression, le générateur de stimulus (102) comprend un générateur de vibrations, afin de stimuler le(s) nerf(s).

6. Le système de la revendication 5, dans lequel pour un stimulus électrique, le générateur de stimulus (102) comprend un générateur de haute tension (22) et un limiteur de courant (24) pour générer le stimulus électrique à appliquer via une matrice de commutation (28), dans lequel le stimulus électrique comprend une série d'impulsions électriques, le module de contrôle de stimulus (112) comprend un module de commande de commutation (14) et un module de commande d'impulsion (18) pour commander la commutation du stimulus électrique, via la matrice de commutation (28), conformément au profil de stimulation, et l'interface de stimulus comprend au moins un dispositif de commutation (40) connecté à la matrice de commutation (28) pour commander le fonctionnement de l'interface de stimulus, dans laquelle une batterie (44) et son chargeur (46) sont connectés à chaque dispositif de commutation (40) associé à chaque interface de stimulation, dans laquelle l'interface de stimulation comprend au moins une paire d'électrodes (36) pour envoyer du courant à travers le tissu de l'utilisateur afin de stimuler le(s) nerf(s) concerné(s), et dans laquelle au moins une paire d'électrodes (36) a une double fonction pour permettre à la paire d'électrodes (36) de fournir et de recevoir du courant électrique, les paires d'électrodes (36) étant disposées dans une configuration prédéterminée pour permettre une stimulation ciblée de zones spécifiques du système nerveux, la paire d'électrodes (36) étant soit parallèle l'une à l'autre pour cibler le nerf ulnaire et/ou le nerf médian, qui longent l'avant-bras, ou soit disposée en croix ou en travers pour cibler le nerf ulnaire et/ou le nerf médian.

7. Le système de la revendication 6, dans lequel, dans le cas d'une série d'impulsions électriques, chaque impulsion de stimulation comprend une impulsion d'une seule fréquence ou une combinaison de deux ou plusieurs impulsions de fréquences identiques ou différentes, dans lequel le profil de stimulation de l'impulsion se situe dans la plage de fréquences comprise entre 0,1 Hz et 1 000 Hz, a une intensité comprise entre 100 uA et 10 mA et une largeur d'impulsion comprise entre 1 uS et 100 mS.1 Hz à 1000 Hz, a une intensité comprise entre 100 uA et 10 mA et une largeur d'impulsion comprise entre 1 uS et 100 mS, la stimulation comprenant la somme de deux fréquences ou plus, y compris une forme d'onde oscillante lente ayant une fréquence d'environ 1 Hz, une forme d'onde oscillante moyenne superposée à la forme d'onde oscillante lente ayant une fréquence d'environ 15 Hz, et une forme d'onde oscillante élevée superposée à l'impulsion de la forme d'onde lente et à la forme d'onde oscillante moyenne, ayant une fréquence comprise entre 30 Hz et 50 Hz.

8. Le système des revendications 6 ou 7, dans lequel le module de traitement de l'entraînement (204) surveille les performances de l'utilisateur pendant l'entraînement et demande au module de contrôle des impulsions (18) de sélectionner un profil d'impulsions de stimulation électrique ou de sélectionner un autre profil d'impulsions de stimulation électrique, si un profil est déjà en cours d'utilisation.

9. Le système de la revendication 8, dans lequel le module de traitement de l'entraînement (204) continue à surveiller la performance de l'utilisateur et, une fois qu'un niveau de performance requis a été atteint, un autre profil d'impulsion de stimulation électrique est sélectionné et appliqué pour maintenir la performance de l'utilisateur.

10. Le système des revendications 8 ou 9, dans lequel le stimulateur nerveux (10, 100) est en communication avec le module de traitement de l'entraînement (204) par l'intermédiaire du module de communication (48, 114), qui est lui-même en communication avec l'ordinateur d'entraînement (200).

11. Le système de l'une quelconque des revendications précédentes, dans lequel le stimulateur nerveux (10, 100) prend la forme d'un stimulateur portable (10, 100), incorporé et/ou logé et/ou intégré et/ou fixé à un dispositif portable, y compris un bracelet de suivi de la condition physique, un fermoir, un patch, une sangle (84), un manchon ou une montre ou une smartwatch (80), dans laquelle la montre utilise un dispositif de fermeture comprenant un corps de fermeture central et incurvé, auquel des parties de bracelet flexibles adjacentes sont fixées de manière articulée, le stimulateur nerveux (10, 100) étant fixé de manière intégrée, ou amovible, au corps de fermeture.

12. Le système de la revendication 11, dans lequel, dans le cas d'une smartwatch (80), la smartwatch (80) comprend un dispositif de montre monté sur un bracelet (84), positionné sur la face dorsale du poignet de l'utilisateur (38) en cours d'utilisation, le dispositif de montre comprenant un dispositif de calcul (82).

13. Méthode non thérapeutique d'utilisation d'un système comprenant un ordinateur d'entraînement (200) et un stimulateur nerveux non invasif (10, 100) pour les nerfs périphériques, y compris les nerfs ulnaire et/ou médian, afin de stimuler les nerfs médian et/ou ulnaire à l'aide d'au moins un stimulus, le stimulateur nerveux (10, 100) comprenant un générateur de stimulus (102), pour générer au moins un stimulus selon un profil de stimulation, un applicateur (34, 104) pour appliquer le stimulus généré au bras, au poignet (38) ou à la main de l'utilisateur (202), et un contrôleur (12, 106) pour commander le fonctionnement du générateur de stimulus (102) et de l'applicateur (34, 104), la méthode comprenant
recevoir au moins un paramètre physiologique associé à un utilisateur (202);
en comparant au moins un paramètre physiologique reçu à un niveau ou à un profil prédéterminé (150, 152);
sélectionner un autre profil de stimulation ou ajuster le profil de stimulation existant en conséquence (154); et
**caractérisée par le fait que** la méthode non thérapeutique comprend en outre:
exécuter une application interactive de formation cognitive sur l'ordinateur d'entraînement (200), avec laquelle l'utilisateur (202) est disposé à interagir, l'ordinateur d'entraînement (200) étant en communication avec le stimulateur nerveux (10, 100);
surveiller l'interaction de l'utilisateur avec l'application interactive de formation cognitive sur l'ordinateur d'entraînement (200); et
coordonner l'application du stimulus avec des activités particulières ou des éléments d'apprentissage auxquels l'utilisateur (202) doit participer et/ou à des moments prédéterminés au cours de la formation.

14. La méthode non thérapeutique de la revendication 13, dans laquelle la méthode non thérapeutique comprend en outre l'étape consistant à continuer à surveiller au moins un paramètre physiologique, et une fois que le niveau ou le profil prédéterminé des paramètres physiologiques est atteint, la méthode comprend la sélection d'un autre profil de stimulation pour maintenir le niveau ou le profil prédéterminé des paramètres physiologiques au moins un paramètre physiologique, ou l'arrêt de la stimulation.

15. La méthode non thérapeutique de la revendication 13 ou 14, dans laquelle la méthode non thérapeutique comprend en outre les étapes suivantes:
surveiller les performances de l'utilisateur au cours de l'entraînement et demander au module de contrôle des stimulus (112) de sélectionner un profil de stimulation ou de sélectionner un autre profil de stimulation, si un profil est déjà utilisé;
continuer à surveiller les performances de l'utilisateur et, une fois qu'un niveau de performance prédéterminé a été atteint, sélectionner un autre profil de stimulation ou arrêter la stimulation.
